(19) Europäisches Patentamt
European Patent Office
Office européen des brevets

(11) EP 1 646 665 B1

(12) # EUROPÄISCHE PATENTSCHRIFT

(45) Veröffentlichungstag und Bekanntmachung des Hinweises auf die Patenterteilung:
**27.12.2006 Patentblatt 2006/52**

(21) Anmeldenummer: **04740089.0**

(22) Anmeldetag: **19.06.2004**

(51) Int Cl.:
***C08F 226/04*** (2006.01) ***C04B 24/26*** (2006.01)
***C04B 24/00*** (2006.01)

(86) Internationale Anmeldenummer:
**PCT/EP2004/006648**

(87) Internationale Veröffentlichungsnummer:
**WO 2005/005500 (20.01.2005 Gazette 2005/03)**

(54) **ALKOXYLIERTE DIALLYLAMINDERIVATE ENTHALTENDE WASSERLÖSLICHE ODER IN WASSER DISPERGIERBARE POLYMERISATE**

POLYMERS THAT ARE SOLUBLE IN WATER OR CAN BE DISPERSED IN WATER AND CONTAIN ALKOXYLATED DIALLYLAMINE DERIVATIVES

POLYMERISATS SOLUBLES OU DISPERSIBLES DANS L'EAU, RENFERMANT DES DERIVES DIALLYLAMINE ALCOXYLES

(84) Benannte Vertragsstaaten:
**AT BE BG CH CY CZ DE DK EE ES FI FR GB GR HU IE IT LI LU MC NL PL PT RO SE SI SK TR**

(30) Priorität: **07.07.2003 DE 10330747**

(43) Veröffentlichungstag der Anmeldung:
**19.04.2006 Patentblatt 2006/16**

(73) Patentinhaber: **BASF Aktiengesellschaft
67056 Ludwigshafen (DE)**

(72) Erfinder:
- **BECKER, Stefan
68167 Mannheim (DE)**
- **CHRISSTOFFELS, Lysander
67117 Limburgerhof (DE)**
- **VÖLKEL, Ludwig
67117 Limburgerhof (DE)**
- **GÖTZ, Thomas
76774 Leimersheim (DE)**
- **MEYER-ROSCHER, Bernd
67434 Neustadt (DE)**
- **HANSCH, Wolfgang
67365 Schwegenheim (DE)**
- **SPILGER, Stefanie
68723 Schwetzingen (DE)**

(56) Entgegenhaltungen:
**US-A- 4 706 755** **US-A- 5 849 853**
**US-B1- 6 242 101**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 646 665 B1

## Beschreibung

Die vorliegende Erfindung betrifft wasserlösliche oder in Wasser dispergierbare Polymerisate enthaltend alkoxylierte Diallylaminderivate, ethylenisch ungesättigte Mono- oder Dicarbonsäuren, deren Anhydride oder Gemische davon sowie gegebenenfalls eines oder mehrere weitere ethylenisch ungesättigte Monomere, Verfahren zu deren Herstellung sowie deren Verwendung als Additive in mineralischen Baustoffen, Waschmitteln oder in kosmetischen Zubereitungen.

US 3,585,148 beschreibt die Copolymerisation von ethoxylierten Diallylaminen und Acrylamid sowie deren Verwendung als Demulgator in dispergierten Öl-in-nicht-öligen kontinuierlichen Phasen.

Die anti-bakterizide Wirkung von polyethoxylierten Diallylaminen für Kontaktlinsen wird in DE 2916698 beansprucht.

Die Verwendung von polyethoxylierten Diallylaminen in Waschmitteln wird in EP 111 965 und EP 112 592 beansprucht. Ebenfalls offenbart ist die Synthese der ethoxylierten Diallylamine aus Diallylamin sowie die Herstellung der Homopolymere. Copolymerisate sind nicht offenbart.

Polycarboxylate mit Polyetherseitenketten bzw. Copolymere aus ungesättigten Säuren und Polyethermakromonomeren und ihre Verwendung als Betonfließmittel, bzw. Zementdispergiermittel sind bekannt.

EP 736 553 beschreibt Copolymere aus ungesättigten Dicarbonsäuren (Maleinsäureanhydrid) und alkoxylierten Alkenylethern (z.B. ethoxylierter Allylalkohol) die bis zu 20 Mol% Acrylsäure- oder Methacrylsäurederivat enthalten können.

DE 19653524 beschreibt wasserlösliche oder wasserdispergierbare Polymerisate, die Carboxylgruppen und Polyalkylenoxidseitenketten enthalten

EP 850 994 beschreibt ein Zement-Additiv, das aus einem ungesättigten Polyalkylenglykolether-Monomer und Maleinsäureanhydrid hergestellt wird.

EP 850 895 beschreibt ein Zement-Dispergiermittel bestehend aus einer Polycarbonsäure mit Polyalkylenoxid-Seitenketten

EP 1 118 598 beschreibt ebenfalls Zement-Dispergiermittel bestehend aus einer Polycarbonsäure mit Polyalkylenoxidseitenketten. Als Monomere werden hier auch ethoxylierter Allylalkohol und ethoxylierter Methallylalkohol genutzt.

WO 01/21542 beschreibt ein Zement-Dispergiermittel erhältlich durch Polymerisation mindestens einer Verbindung (A) vom Typ $CH_2=CR_1CH_2O(R_2O)_mR_3$, mit $R_1$ Wasserstoff oder Methyl; $R_2$ $C_2$-$C_3$-Alkylen; $R_3$ Wasserstoff oder Methyl und m ist eine ganze Zahl zwischen 1 und 300) und mindestens einer Verbindung (B) $CH_2=CR_4COO(R_sO)_nR_6$, mit $R_4$ Wasserstoff oder Methyl; $R_5$ $C_2$-$_3$-Alkylen; $R_6$ Wasserstoff oder Methyl und n ist eine ganze Zahl zwischen 1 und 300) und (C) mindestens einer Verbindung ausgewählt aus der Gruppe Maleinsäure oder Maleinsäureanhydrid und (D) mindestens einer Verbindung ausgewählt aus der Gruppe Acrylsäure, Methacrylsäure oder Itaconsäure und anschließender Neutralisation des Copolymers.

Zusammenfassend kann gesagt werden, dass die aus dem Stand der Technik bekannten Additive für die erfindungsgemäßen Verwendungen noch der Verbesserung bedürfen.

Insbesondere ist die verflüssigende Wirkung der Additive in mineralischen Baustoffen bei niedrigen Wasser/Bindemittel-Verhältnissen in der Regel noch nicht ausreichend oder bleibt nur über eine kurze Zeitspanne erhalten. Eine höhere Dosierung des Fließmittels kann diesen Mangel zwar teilweise ausgleichen, dies hat aber in aller Regel neben der Unwirtschaftlichkeit einer solchen Vorgehensweise erhebliche Einbußen bei der erreichbaren mechanischen Festigkeit oder zumindest nicht akzeptable Verzögerungen der Abbindegeschwindigkeiten zur Folge.

Der vorliegenden Erfindung lag somit die Aufgabe zugrunde, Additive, insbesondere für mineralische Baustoffe zur Verfügung zu stellen, die hinsichtlich ihrer verflüssigenden Wirkung Vorteile gegenüber den bekannten Additiven für mineralische Baustoffe aufweisen.

Überraschenderweise wurde nun gefunden, dass wasserlösliche oder in Wasser dispergierbare Polymerisate enthaltend

(a) mindestens ein alkoxyliertes Diallylaminderivat (Monomer A)
(b) mindestens eine ethylenisch ungesättigte Mono- oder Dicarbonsäure, deren Anhydride oder Gemische davon (Monomer B) sowie
(c) gegebenenfalls eines oder mehrere weitere ethylenisch ungesättigte Monomere C

vorteilhafte Eigenschaften als Additive für Waschmittel, in kosmetischen Mitteln und insbesondere in mineralischen Baustoffen aufweisen.

Die erfindungsgemäßen Verbindungen weisen trotz ausgezeichneter verflüssigender Wirkung auf zementäre Baustoffe weniger Nachteile im Abbindeverhalten oder hinsichtlich der Festigkeit der abgebundenen Baustoffe auf.

Gegenstand der Erfindung ist weiterhin die Verwendung der Polymerisate in Waschmitteln, kosmetischen

Mitteln, in mineralischen Baustoffen, sowie mineralische Baustoffe, Waschmittel oder kosmetische Mittel enthaltend die erfindungsgemäßen Polymerisate sowie Verfahren zu ihrer Herstellung.

In einer bevorzugten Ausführungsform wird als Monomer A mindestens eine Verbindung der allgemeinen Formel I eingesetzt

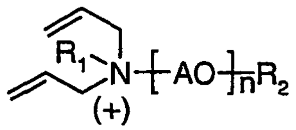

(I)

wobei

| | |
|---|---|
| AO | ein $C_1$-$C_{12}$-Alkylenoxid, Styroloxid oder ein Gemisch von zwei oder mehr Arten hiervon bedeutet, wobei die zwei oder mehr Arten entweder in Blockform oder in statistischer Form aneinander gefügt sein können, |
| n | eine ganze Zahl von 2 bis 200 |
| $R_1$ | Wasserstoff, $C_1$-$C_{20}$-Alkyl, $C_5$-$C_{10}$-Cycloalkyl oder einen gegebenenfalls substituierten Benzylrest bedeutet und |
| $R_2$ | Wasserstoff, $C_1$-$C_{30}$-Alkyl, $C_5$-$C_8$-Cycloalkyl, $C_6$-$C_{20}$-Aryl, $C_1$-$C_{30}$-Alkanoyl, $C_7$-$C_{21}$-Aroyl, Schwefelsäure (halb)ester, Phosphorsäureester, NR'R", NR'R"R"'$^{3+}$bedeutet und |
| R', R", R"' | jeweils unabhängig voneinander gleich oder verschieden sein können und Wasserstoff, einen geradkettig oder verzweigten $C_1$-$C_{20}$-Alkyl- oder einen geradkettig oder verzweigten $C_1$-$C_{20}$-Hydroxyalkylrest bedeuten. |

In einer ebenfalls bevorzugten Ausführungsform wird als Monomer B mindestens eine Verbindung der allgemeinen Formel II, oder deren Anhydride eingesetzt

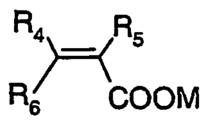

(II)

wobei

| | |
|---|---|
| $R_4$, $R_5$ | unabhängig voneinander entweder gleich oder verschieden sein können und Wasserstoff oder $C_1$-$C_6$-Alkyl bedeuten, |
| $R_6$ | Wasserstoff, $C_1$-$C_6$-Alkyl oder eine Gruppe COOM bedeutet und |
| M | Wasserstoff, ein ein- oder zweiwertiges Metallion, Ammonium oder ein organisches Ammoniumion bedeutet. |

Unter mineralischen Baustoffen sind Zubereitungen zu verstehen, die als wesentliche Bestandteile mineralische Bindemittel wie Kalk, Gips und/oder insbesondere Zement sowie als Zuschläge dienende Sande, Kiese, gebrochene Gesteine oder sonstige Füllstoffe z.B. natürliche oder synthetische Fasem enthalten. Die mineralischen Baustoffe werden in der Regel durch Zusammenmischung der mineralischen Bindemittel und der Zuschläge zusammen mit Wasser in eine gebrauchsfertige Zubereitung überführt, die, wenn sie sich selbst überlassen ist, mit der Zeit an der Luft oder auch unter Wasser steinartig aushärtet

Anmerkung: Unter Wasser geht bei Zement, aber nicht bei Kalk und nur schlecht bei Gips.

Unter $C_1$-$C_{12}$-Alkylenoxiden versteht man beispielsweise Ethylenoxid, Propylenoxid, 1-Butylenoxid, Isomere des Butylenoxid, höhere Alkylenoxide, wie Dodecenoxid, Styroloxid sowie Mischungen der Oxide in beliebiger Reihenfolge, wobei der Ethylenoxidgehalt mindestens 40 % betragen sollte . Bevorzugt bedeutet Alkylenoxid Ethylenoxid oder Mischungen aus Ethylenoxid und Propylenoxid.

n bedeutet eine ganze Zahl von 2 bis 200, bevorzugt von 5 bis 150, insbesonders bevorzugt von 10 bis 100.

Unter einem $C_1$-$C_{20}$-Alkylrest versteht man lineare oder verzweigte gesättigte Kohlenwasserstoffketten mit bis zu 20, vorzugsweise mit 1 bis 10 Kohlenstoffatomen, z.B. Methyl, Ethyl, n-Propyl, i-Propyl, n-Butyl, t-Butyl, n-Pentyl, Neopentyl, n-Hexyl, 2-Ethylhexyl, n-Octyl, 1-Decyl, 1-Dodecyl etc., vorzugsweise Methyl, Ethyl, n-Propyl oder i-Propyl.

Unter einem $C_5$-$C_8$-Cycloalkylrest versteht man einen cycloaliphatischen Rest mit 5 bis 8 Kohlenstoffatomen, ausgewählt unter Cyclopentyl, Cyclohexyl, Cycloheptyl oder Cyclooctyl, die gegebenenfalls mit 1,2,3 oder 4 $C_1$-$C_4$-Al-

kylgruppen substituiert sein können.

$C_6$-$C_{20}$-Aryl steht für Arylgruppen, die über eine Alkyleneinheit gebunden sind und die 6 bis 20 Kohlenstoffatome aufweisen können, z.B. Benzyl, Phenyl, oder Ethylphenyl.

$C_1$-$C_{30}$ Alkanoyl steht für einen Rest, der sich von einer aliphatischen Carbonsäure ableitet und umfasst somit neben Formyl solche Alkylreste, die über eine Carbonylgruppe gebunden sind.

$C_7$-$C_{21}$- Aroyl entspricht $C_7$-$C_{21}$-Arylcarbonyl und steht für einen Arylrest, der über eine Carbonylgruppe gebunden ist und leitet sich somit von Derivaten der Benzoesäure und der Naphtoesäure ab.

Unter einem ein- oder zweiwertigen Metallion versteht man die Kationen der Elemente der ersten und zweiten Hauptgruppe des Periodensystems, d.h. $Li^+$, $Na^+$, $K^+$, $Rb^+$, $Cs^+$, $Be^{2+}$, $Mg^{2+}$, $Ca^{2+}$, $Sr^{2+}$ und $Ba^{2+}$ sowie $Ag^+$, $Fe^{2+}$, $Co^{2+}$, $Ni^{2+}$, $Cu^{2+}$, $Zn^{2+}$, $Cd^{2+}$, $Sn^{2+}$, $Pb^{2+}$, $Ce^{2+}$. Bevorzugt sind die Kationen der Alkali und Erdalkalimetalle $Li^+$, $Na^+$, $K^+$, $Rb^+$, $Cs^+$, $Be^{2+}$, $Mg^{2+}$, $Ca^{2+}$, $Sr^{2+}$ und $Ba^{2+}$ sowie $Zn^{2+}$. Besonders bevorzugt $Li^+$, $Na^+$, $K^+$, $Mg^{2+}$, $Ca^{2+}$ und $Zn^{2+}$.

Eine organisches Ammoniumion bedeutet ein einwertiges Ion, das durch Protonierung eines Mono-, Di- oder Trialkylamin oder eines Mono-, Di-oder Trialkanolamins mit 1 - 10 Kohlenstoffatomen entsteht. Beispiele für Mono-, Di- und Trialkylamine sind Methylamin, Ethylamin, n-Propylamin, i-Propylamin, Dimethylamin, Diethylamin, Di-n-propylamin, Di-i-propylamin, Trimethylamin, Triethylamin. Beispiele für Mono-, Di- und Trialkanolamine sind 2-Aminoethanol, Diethanolamin, Triethanolamin, Triisopropanolamin.

$R_1$ bedeutet vorzugsweise Wasserstoff, Methyl, Ethyl, n-Propyl, n-Butyl oder Benzyl, besonders bevorzugt Wasserstoff oder Methyl.

$R_2$ bedeutet vorzugsweise Wasserstoff, Methyl, Ethyl, n-Propyl, n-Butyl oder Phenyl, besonders bevorzugt Wasserstoff oder Methyl.

$R_4$ und $R_5$ bedeuten vorzugsweise Wasserstoff oder Methyl

$R_6$ bedeutet vorzugsweise Wasserstoff , Methyl oder eine Gruppe COOM.

M bedeutet vorzugsweise Wasserstoff oder ein einwertiges Metallion.

R', R'', R''' bedeuten vorzugsweise Wasserstoff, Methyl, Ethyl oder 2-Hydroxyethyl.

Als Monomere A werden bevorzugt alkoxylierte Diallylaminamine mit 2 -100 mol Alkylenoxid eingesetzt, die als weiteren Rest $R_1$ bevorzugt Wasserstoff oder Methyl tragen. Bevorzugte Alkylenoxide sind dabei Ethylenoxid oder Propylenoxid, die allein, in statistischer oder Blockreihenfolge im Monomer A vorliegen können.

Als Monomere B werden bevorzugt monoethylenisch ungesättigte $C_3$-$C_6$-Monocarbonsäuren wie Acrylsäure, Methacrylsäure, Crotonsäure, Isocrotonsäure oder 2-Ethylpropensäure eingesetzt, ethylenisch ungesättigte $C_4$-$C_6$-Dicarbonsäuren wie Maleinsäure, Furnarsäure, Itaconsäure oder deren Anyhdride, wie beispielsweise Maleinsäureanhydrid oder deren Natrium-, Kalium- oder Ammoniumsalze.

Neben den Monomeren A und B kann das Polymerisat gegebenenfalls noch Monomere C enthalten. Als Monomere C können beispielsweise $C_1$-$C_8$-Alkylester oder $C_1$-$C_4$ Hydroxyalkylester der Acrylsäure, Methacrylsäure oder Maleinsäure oder Ester von mit 2 bis 50 mol Ethylenoxid, Propylenoxid, Butylenoxid oder Mischungen davon, alkoxylierten $C_1$-$C_{18}$-Alkoholen mit Acrylsäure, Methacrylsäure oder Maleinsäure, z.B. Hydroxyethylacrylat, Hydroxyethylmethacrylat, Hydroxypropyl(meth) acrylat, Butyl(meth)acrylat eingesetzt werden.

Das molare Verhältnis der Monomeren A zu den Monomeren B beträgt 1 : 1 bis 1 : 6, bevorzugt 1 : 2 bis 2 : 5.

Bevorzugte Polymerisate enthalten 1 - 70 Mol% Monomer A, 10 - 99 Mol-% Monomer B und 0 - 50 Mol% Monomer C.

Die Herstellung der Alkylenoxide kann z.B. durch Alkoxylierung von Diallylamin in mehreren Schritten erfolgen. In einem ersten Schritt wird Diallylamin in Gegenwart eines Lösungsmittels oder auch pur mit mindestens einem Äquivalent Alkylenoxid umgesetzt. Die so erhaltene Vorstufe wird in Gegenwart eines Katalysators weiter mit Alkylenoxid umgesetzt, wobei alle aus dem Stand der Technik für die Polymerisation von Alkylenoxiden bekannten Katalysatoren verwendet werden können - wobei alle aus dem Stand der Technik für die Polymerisation von Alkylenoxiden bekannten und mit Aminen kompatiblen Katalysatoren in Frage kommen. Ein Überblick über einige Katalysatoren wird z.B. gegeben in F.E. Bailey, Jr, J.V. Koleske, Alkylene Oxides and their Polymers, NY and Basel 1991, S. 35 ff. Besonders bevorzugt werden basische Katalysatoren wie NaOH, KOH, CsOH, KotBu. NaOMe oder Mischungen der Basen mit Kronenethern verwendet.

Das Additionsprodukt aus Alkylenoxiden und Diallylamin kann weiter funktionalisiert werden. Beispielsweise kann mit Alkylierungsmitteln quaterniert werden, die OH-Gruppen können in Sulfate, Sulfonate, Phosphate oder Phosphonate überführt werden. Es resultieren dann kationische, anionische oder betainische Strukturen.

Die Polymerisate können nach üblichen Polymerisationsverfahren als Substanzpolymerisation, Lösungspolymerisation und bei schlechter Löslichkeit der Monomere auch als Emulsions, Dispersions- oder Suspensionspolymerisation durchgeführt werden. Ebenfalls ist es möglich, bei hinreichend schlechter Löslichkeit des Polymerisats im Reaktionsgemisch die Polymerisation als Fällungspolymerisation durchzuführen.

Bei den genannten Polymerisationsverfahren wird bevorzugt unter Ausschluss von Sauerstoff, vorzugsweise in einem Stickstoffstrom gearbeitet. Für alle Polymerisationsmethoden werden die üblichen Apparaturen verwendet, z. B. Rührkessel, Rührkesselkaskaden, Autoklaven, Rohrreaktoren und Kneter. Bevorzugt sind die Methoden der Lösungs- und Emulsionspolymerisation. Erfolgt die Herstellung der erfindungsgemäßen Polymerisate durch radikalische, wässrige Emulsionspolymerisation, empfiehlt es sich, dem Reaktionsmedium Tenside oder Schutzkolloide zuzusetzen. Eine Zusammenstellung geeigneter Emulgatoren und Schutzkolloide findet sich beispielsweise in Houben Weyl, Methoden der organischen Chemie, Band XIV/1 Makromolekulare Stoffe, Georg Thieme Verlag, Stuttgart 1961, S. 411 ff.

Die Polymerisation kann in Lösungs- oder Verdünnungsmitteln, wie z.B. Toluol, o-Xylol, p-Xylol, Cumol, Chlorbenzol, Ethylbenzol, technischen Mischungen von Alkylaromaten, Cyclohexan, technischen Aliphatenmischungen, Aceton, Cyclohexanon, Tetrahydrofuran, Dioxan, Glykolen und Glykolderivaten, Polyalkylenglykolen und deren Derivate, Diethylether, tert.-Butylmethylether, Essigsäuremethylester, Isopropanol, Ethanol, Wasser oder Mischungen wie z.B. Isopropanol/Wasser-Mischungen ausgeführt werden. Vorzugsweise wird als Lösungs- oder Verdünnungsmittel Wasser, gegebenenfalls mit Anteilen bis zu 60 Gew.-% an Alkoholen oder Glykolen verwendet. Besonders bevorzugt wird Wasser eingesetzt.

Die Polymerisation kann bei Temperaturen von 20 bis 300, vorzugsweise von 40 bis 150°C durchgeführt werden. Je nach Wahl der Polymerisationsbedingungen lassen sich gewichtsmittlere Molekulargewichte ($M_w$) z.B. von 1000 bis 1 00 000, bevorzugt von 5000 - 50000 einstellen. $M_w$ wird bestimmt durch Gelpermeationschromatographie.

Die Polymerisation wird vorzugsweise in Gegenwart von Radikale bildenden Verbindungen durchgeführt. Man benötigt von diesen Verbindungen bis zu 30, vorzugsweise 0,05 bis 15, insbesonders bevorzugt 0,2 bis 8 Gew.-%, bezogen auf die bei der Polymerisation eingesetzten Monomeren. Bei mehrkomponentigen Initiatorsystemen (z.B. Redox-Initiatorsystemen) beziehen sich die vorstehenden Gewichtsangaben auf die Summe der Komponenten.

Geeignete Polymeriationsinitiatoren sind beispielsweise Peroxide, Hydroperoxide, Peroxodisulfate, Percarbonate, Peroxidester, Wasserstoffperoxid und Azoverbindungen. Beispiele für Initiatoren, die wasserlöslich oder auch wasserunlöslich sein können, sind Wasserstoffperoxid, Dibenzoylperoxid, Dicyclohexylperoxidicarbonat, Dilauroylperoxid, Methylethylketonperoxid, Di-tert.-Butylhydroperoxid, Acetylacetonperoxid, tert.-Butylhydroperoxid, Cumolhydroperoxid, tert.-Butylperneodecanoat, tert.-Amylperpivalat, tert.-Butylperpivalat, tert.-Butylperbenzoat, Lithium-, Natrium-, Kalium- und Ammoniumperoxodisulfat und Azodiisobutyronitril.

Die Initiatoren können allein oder in Mischung untereinander angewendet werden, z.B. Mischungen aus Wasserstoffperoxid und Natriumperoxodisulfat. Für die Polymerisation in wässrigem Medium werden bevorzugt wasserlösliche Initiatoren eingesetzt.

Auch die bekannten Redox-Initiatorsysteme können als Polymerisationsinitiatoren verwendet werden. Solche Redox-Initiatorsysteme enthalten mindestens eine peroxidhaltige Verbindung in Kombination mit einem Redox-Coinitiator z.B. reduzierend wirkende Schwefelverbindungen, beispielsweise Bisulfite, Sulfite, Thiosulfate, Dithionite und Tetrathionate von Alkalimetallen und Ammoniumverbindungen. So kann man Kombinationen von Peroxodisulfaten mit Alkalimetall- oder Ammoniumhydrogensulfiten einsetzen, z.B. Ammoniumperoxodisulfat und Ammoniumdisulfit. Die Menge der peroxidhaltigen Verbindung zum Redox-Coinitiator beträgt 30 : 1 bis 0,05 : 1.

In Kombination mit den Initiatoren bzw. den Redoxinitiatorsystemen können zusätzlich Übergangsmetallkatalysatoren eingesetzt werden, z.B. Salze von Eisen, Kobalt, Nickel, Kupfer, Vanadium und Mangan. Geeignete Salze sind z.B. Eisen (II).sulfat, Kobalt (II)chlorid, Nickel(II)sulfat, oder Kupfer(I)chlorid. Bezogen auf die Monomeren wird das reduzierend wirkende Übergansmetallsalz in einer Konzentration von 0,1 ppm bis 1000 ppm eingesetzt. So kann man Kombinationen von Wasserstoffperoxid mit Eisen(II)-Salzen einsetzen, wie beispielsweise 0,5 bis 30 % Wasserstoffperoxid und 0,1 bis 500 ppm Mohrsches Salz.

Auch bei der Polymerisation in organischen Lösungsmitteln können in Kombination mit den oben genannten Initiatoren Redox-Coinitiatoren und/oder Übergangsmetallkatalysatoren mitverwendet werden, z.B. Benzoin, Dimethylanilin, Ascorbinsäure sowie organisch lösliche Komplexe von Schwermetallen, wie Kupfer, Cobalt, Eisen, Mangan, Nickel und Chrom. Die üblicherweise verwendeten Mengen an Redox Coinitiatoren bzw. Übergansmetallkatalysatoren betragen etwa 0,1 bis 1000 ppm, bezogen auf die eingesetzten Mengen an Monomeren.

Um das mittlere Molekulargewicht der Polymerisate zu kontrollieren, ist es oft zweckmäßig, die Copolymerisation in Gegenwart von Reglern durchzuführen. Hierfür können übliche Regler verwendet werden, wie beispielsweise organische SH-Gruppen enthaltende Verbindungen, wie 2-Mercaptoethanol, 2-Mercaptopropanol, 3-Mercaptopropionsäure, Cystein, N-Acetylcystein, aber auch Natriumhypophosphit oder Natriumhydrogensulfit. Die Polymerisationsregler werden im allgemeinen in Mengen von 0,1 bis 10 Gew.-%, bezogen auf die Monomeren eingesetzt. Auch durch die Wahl des geeigneten Lösungsmittels kann auf das mittlere Molekulargewicht Einfluss genommen werden. So führt die Polymerisation in Gegenwart von Verdünnungsmitteln mit benzylischen H-Atomen zu einer Verringerung des mittleren

Molekulargewichtes durch Kettenübertragung.

Um das Molekulargewicht der Polymerisate zu erhöhen, kann es zweckmäßig sein, die Copolymerisation in Gegenwart von geringen Mengen Vernetzern durchzuführen. Hierfür können übliche Vernetzer wie Bis(acrylsäurester) von Diolen, wie Ethylenglykol, Diethylenglykolbisacrylat, Trietylenglykol oder Polyethylenglykol in einer Menge von 0,01 - 5 % bezogen auf die Monomere eingesetzt werden.

Wird das Polymerisat nach dem Verfahren einer Lösungspolymerisation in Wasser gewonnen, so ist üblicherweise keine Abtrennung des Lösungsmittels notwendig. Besteht dennoch der Wunsch, das Polymerisat zu isolieren, kann z.B. eine Sprühtrocknung durchgeführt werden.

Wird das Polymerisat nach der Methode einer Lösungs-, Fällungs- oder Suspensionspolymerisation in einem wasserdampfflüchtigen Lösungsmittel oder Lösungsmittelgemisch hergestellt, so kann das Lösungsmittel durch Einleiten von Wasserdampf abgetrennt werden, um so zu einer wässrigen Lösung oder Dispersion zu gelangen. Das Polymerisat kann von dem organischen Verdünnungsmittel auch durch einen Trocknungsprozess abgetrennt werden.

Bevorzugt liegen die Polymerisate in Form einer wässrigen Dispersion oder Lösung mit Feststoffgehalten von vorzugsweise 10 bis 80 Gew.-%, insbesondere 30 bis 65 Gew.-% vor. Die K- Werte der Polymerisate liegen bevorzugt im Bereich von 20 - 45.

Die erfindungsgemäßen Polymerisate eignen sich hervorragend als Zusatzmittel für Zementmischungen, wie Beton oder Mörtel. Unter Zement ist beispielsweise Portlandzement, Tonerdezement oder vermischter Zement, wie beispielsweise Puzzolanzement, Schlackenzement oder andere Typen zu verstehen. Portlandzement wird bevorzugt. Die Copolymere werden in einer Menge von 0,01 bis 10 Gew.-%, vorzugsweise 0,05 bis 3 Gew.-%, bezogen auf das Gesamtgewicht des Zementes eingesetzt.

Die Polymerisate können in fester Form, die durch Trocknung, beispielsweise durch Sprühtrocknung von Polymerlösungen oder Dispersionen, wie sie bei der Polymerisation anfallen, erhältlich ist, zu der gebrauchsfertigen Zubereitung des mineralischen Baustoffs gegeben werden. Auch ist es denkbar, die Copolymerisate mit dem mineralischen Bindemittel zu formulieren und hieraus die gebrauchsfertigen Zubereitungen des mineralischen Baustoffs zu bereiten. Vorzugsweise wird das Copolymer in flüssiger, d.h. gelöster emulgierter oder suspendierter Form, beispielsweise in Form der Polymerisationslösung, bei der Zubereitung des mineralischen Baustoffs eingesetzt.

Für die Anwendung in Beton oder Mörtel kann es vorteilhaft sein, Polymere einzusetzen, die erst in Gegenwart des alkalischen Betons oder Mörtels in eine wasserlösliche und damit wirksame Form übergehen, wie z.B. Carbonsäure- oder Carbonsäureanhydridstrukturen. Die langsame Freigabe des wirksamen Polymeren hat eine länger anhaltende Wirksamkeit zur Folge.

Die erfindungsgemäßen Polymeren können auch in Kombination mit den bekannten Betonfließmitteln und/ oder Betonverflüssigern auf Basis von Naphthalinformaldehyd-Kondensat-Sulfonat, Melaminformaldehyd-Kondensaten-Sulfonat, Phenolsulfonsäure-Formaldehydkondensat, Ligninsulfonaten und Gluconaten eingesetzt werden.

Weiterhin können sie zusammen mit Cellulosen, z.B. Alkyl- bzw. Hydroxyalkylcellulosen, Stärken oder Stärkederivaten eingesetzt werden. Auch können sie in Kombinaton mit hochmolekularen Polethylenoxiden (Mw 100 000 - 8 000 000) verwendet werden.

Weiterhin können Additive wie Luftporenbildner, Expansionsmittel, Hydrophobiermittel, Abbindeverzögerer, Abbindebeschleuniger, Frostschutzmittel, Dichtungsmittel, Pigmente, Korrosionsinhibitoren, Fließmittel, Einpreßhilfen, Stabilisierer oder Mikrohohlkugeln zugemischt werden. Solche Additive sind beispielsweise in der EN 934 beschrieben.

Grundsätzlich können die erfindungsgemäßen Polymerisate auch zusammen mit filmbildenden Polymerisaten verwendet werden. Hierunter sind solche Polymerisate zu verstehen deren Glasübergangstemperatur ≤ 65°C, vorzugsweise ≤ 50°C, besonders bevorzugt ≤ 25°C und ganz besonders bevorzugt ≤ 0°C ist. Anhand der von Fox (T.G. Fox, Bull. Am. Phys. Soc. (Ser.II) 1, 1956, 123 aufgestellten Beziehung zwischen Glasübergangstemperatur von Homopolymerisaten und der Glasüberganstemperatur von Copolymeren ist der Fachmann in der Lage, geeignete Polymere auszuwählen.

Weiterhin ist es oftmals von Vorteil, wenn man die erfindungsgemäßen Polymerisate gemeinsam mit Antischaummitteln verendet. Hierdurch wird verhindert, dass beim Zubereiten der gebrauchsfertigen mineralischen Baustoffe zu viel Luft in Form von Luftporen in den Beton eingetragen werden, welche die Festigkeit des abgebundenen mineralischen Baustoffs herabsetzen würden. Geeignete Antischaummittel umfassen insbesondere Antimschaummittel auf Polyalyklenoxidbasis, Trialkylphosphate, wie Tributylphosphat, und Silikon-basierende Entschäumer. Ebenfalls geeignet sind die Ethoxylierungsprodukte und die Propoxylierungsprodukte von Alkoholen mit 10 bis 20 Kohlenstoffatomen. Ebenfalls geeignet sind die Diester von Alkylenglykolen bzw. Polyalkylenglykolen sowie weitere übliche Antischaummittel. Derartige Antischaummittel werden üblicherweise in Mengen von 0,05 Gew.-% bis 10 Gew.-% und vorzugsweise von 0,5 bis 5 Gew.-%, bezogen auf die Polymerisate verwendet.

Die Antischaummittel können auf verschieden Weise mit dem Polymer kombiniert werden. Liegt das Polymer beispielsweise als wässrige Lösung vor, kann das Antischaummittel fest oder gelöst zu der Polymerlösung zugegeben werden. Ist das Antischaummittel in der wässrigen Polymerlösung nicht löslich, dann können Emulgatoren oder Schutzkolloide zu seiner Stabilisierung zugesetzt werden.

Liegt das erfindungsgemäße Polymerisat in Form eines Feststoffes, wie er z.B. aus einer Sprühtrocknung oder Sprühwirbelschichtgranulierung hervorgeht, so kann das Antischaummittel als Feststoff zugemischt werden oder aber beim Sprühtrocknungsprozess oder Sprühgranulierungsprozess gemeinsam mit dem Polymeren konfektioniert werden.
Die nachfolgenden Beispiele sollen die Erfindung näher erläutern, ohne sie jedoch darauf einzuschränken:

Beispiele

I. Analytik

Bestimmung des mittleren Molekulargewichtes

Die Bestimmung des gewichtsmittleren Molekulargewichtes erfolgte durch Gel-Permeations- Chromatographie (=GPC) mit wässrigen Elutionsmitteln. Die GPC wurde mit einer Gerätekombination der Firma Agilent ( Serie 1100) durchgeführt. Hierzu gehören:

| | |
|---|---|
| Begaser | Modell G 1322 A |
| Isokratische Pumpe | Modell G 1310 A |
| Autosampler | Modell G 1313 A |
| Säulenofen | Modell G 1316 A |
| Steuermodul | Modell G 1323. B |
| Differentialrefraktometer | Modell G 1362 A |

Als Eluent dient im Falle von in Wasser gelösten Polymeren ein 0,08 mol/L TRIS-Puffer (pH=7,0) in destilliertem Wasser + 0,15 mol/L Chlorid-Ionen aus NaCl und HCl.
Die Trennung fand in einer Trennsäulenkombination statt. Verwendet werden die Säulen Nr. 787 und 788 (je 8 x 30 mm) der Firma PSS mit Trennmaterial GRAL BIO linear. Die Durchflussgeschwindigkeit betrug 0,8 mUmin bei einer Säulen-Temperatur von 23°C.
Die Kalibrierung erfolgt mit Polyethylenoxid-Standards der Fa. PPS mit Molekulargewichten von M = 194 - 1700000 [mol/g].

Bestimmung des K-Wertes

Die K-Werte der wässrigen Natriumsalzlösungen der Copolymerisate wurden nach H. Fikentscher, Cellulose-Chemie, Band 13, 58-64 und 71-74 (1932) in wässriger Lösung bei einem pH-Wert von 7, einer Temperatur von 25°C und eienr Polymerkonzentration des Natriumsalzes des Copolymerisates von 1 Gew.-% bestimmt.

Bestimmung des Feststoffgehaltes

In ein Alu-Schälchen wird eine definierte Menge der Probe (ca. 0,5 -1 g) eingewogen (Einwaage). Die Probe wird unter einer IR-Lampe (160 Volt) für 30 Minuten getrocknet.
Danach wird erneut die Masse der Probe bestimmt (Auswaage). Der prozentuale Feststoffgehalt (FG) errechnet sich wie folgt:

$$FG = Auswaage \times 100/\ Einwaage\ [Gew.-\%]$$

1. Herstellung der Reaktivalkoxylate:

Beispiel A: Diallylamin + 21 EO

In einem 20 L Stahlreaktor mit Mantelkühlung, Oxiddosierung und Innenthermometer wurden 2,471 kg Diallylamin und 0,126 kg demineralisiertes Wasser vorgelegt. Der Reaktor wurde kurz evakuiert und anschließend bei 25°C mit Stickstoff ein Druck von 15,4 bar aufgebaut. Nach 50 Minuten wurde auf 3 bar entspannt und auf 80°C erwärmt. Nun wurde innerhalb von 80 Minuten 1,120 kg Ethylenoxid so zudosiert, dass der Druck zwischen 2,8 und 4,3 bar gehalten wurde und die Temperatur 95°C nicht überstieg. Nach Dosierung des Ethylenoxid ließ man 120 Minuten nachrühren und kühlte dann auf 50°C ab. 1,217 kg wurden aus dem Reaktor abgelassen. Zu dem verbleibenden Material

wurden 0,1463 kg einer 45% wässrigen KOH-Lösung zugefügt. Die Temperatur wurde auf 103°C erhöht und bei einem Druck von <10 mbar entwässert. Anschließend wurde mit Stickstoff ein Druck von 2 bar aufgebaut und auf 122°C erwärmt und innerhalb von 1310 Minuten 14,817 kg Ethylenoxid zugegast wobei der Druck zwischen 2 und 5,5 bar gehalten wurde und die Temperatur 135°C nicht überstieg. Die Dosierung wurde nach 240 Minuten unterbrochen, bei 118°C weitergerührt, das restliche Oxid innerhalb von 110 Minuten dosiert und 129 Minuten bei dieser Temperatur weitergerührt. Es wurde auf 80°C abgekühlt und 10,36 kg wurden aus dem Reaktor abgelassen. Das Produkt hatte eine OH-Zahl von 62,9 mg KOH/g.

Beispiel B: Diallylamin + 40 EO

Das nach Beispiel 1 im Reaktor verbliebene Produkt wurde im selben Reaktor auf 86°C erwärmt und mit Stickstoff inertisiert und ein Druck von 2 bar aufgebaut. Anschließend wurde auf 115°C erwärmt und innerhalb von 240 Minuten 6,964 kg Ethylenoxid so zugegast, dass die Temperatur 130°C nicht überstieg und der Druck zwischen 2 und 5,8 bar blieb. Nach beendeter Zudosierung wurde noch 120 Minuten bei 117°C nachgerührt und 13,24 kg Produkt aus dem Reaktor ausgefüllt. Das Produkt hatte eine OH-Zahl von 32,03 mg KOH/g.

Beispiel C: Diallylamin + 80 EO

3,574 kg des nach Beispiel 1 hergestellten Produktes wurden in dem in Beispiel 1 verwendeten Reaktor mit 0,04464 kg einer 45 %igen wässrigen KOH-Lösung versetzt und analog Beispiel 2 mit 10,105 kg Ethylenoxid umgesetzt. Es wurden 13,51 kg Reaktoraustrag erhalten mit einer OH-Zahl von 24,13 mg KOH/g.

Quaternierung

Beispiel 1

Diallylamin + 20EO quaterniert

In einem 1-L-Glasreaktor mit Wasserbadheizung, Ankerrührer, Innenthermometer, Rückflußkühler und Tropftrichter wurden 548,18 g Diallylamin + 20EO bei 60°C geschmolzen. Zu dieser Schmelze tropfte man gleichmäßig innerhalb einer Stunde 67,10 g Dimethylsulfat. Nach beendeter Zugabe wurde noch 2,5 Stunden bei 60°C gerührt, um die Reaktion zu vervollständigen.

Beispiel 2

Diallylamin + 40EO quaterniert

In einem 500-ml-Vierhalskolben mit Wasserbadheizung, Ankerrührer, Innenthermometer, Rückflußkühler und Tropftrichter wurden 260,00 g Diallylamin + 40EO bei 65°C geschmolzen. Zu dieser Schmelze tropfte man gleichmäßig innerhalb einer Stunde 16,74 g Dimethylsulfat. Nach beendeter Zugabe wurde noch 2 Stunden bei 65°C gerührt, um die Reaktion zu vervollständigen.

II. Herstellvorschriften der Copolymere

Beispiel 1:

In einen 1 l Glasreaktor mit Ankerrührer, Thermometer, Stickstoff-Einleitung, Rückflusskühler und Tropftrichter wird 306,21 g Wasser vorgelegt und aufgeheizt bis 100°C. Zum Inertisieren Stickstoff in den Reaktor einleiten. Bei Rückfluss 192g Diallylamin + 20EO gelöst in 48 g Wasser und 56,57 g Acrylsäure in 5 Stunden zutropfen sowie 18,65 g einer 20 %igen Na-peroxodisulfatlösung und 18,65 g einer 20 %igen Na-hypophosphitlösung in 5,25 Stunden. Um die Copolymerisation zu vervollständigen 1 Stunde nachpolymerisieren, anschließend kühlen und mit 50 %iger Natronlauge neutralisieren.

Beispiel 2:

In einen 1 l Glasreaktor mit Ankerrührer, Thermometer, Stickstoff-Einleitung, Rückflusskühler und Tropftrichter wird 200 g Diallylamin + 20EO gelöst in 294,91 g Wasser vorgelegt und aufgeheizt bis 100°C. Zum Inertisieren Stickstoff in den Reaktor einleiten. Bei Rückfluss 58,93 g Acrylsäure in 5 Stunden zutropfen sowie 19,4 g einer 20 %igen Na-peroxodisulfatlösung und 19,4 g einer 20 %igen Na-hypophosphitlösung in 5,25 Stunden. Um die Copolymerisation zu vervollständigen 1 Stunde nachpolymerisieren, anschließend kühlen und mit 50 %iger Natronlauge neutralisieren.

Beispiel 3:

In einen 1 l Glasreaktor mit Ankerrührer, Thermometer, Stickstoff-Einleitung, Rückflusskühler und Tropftrichter wird 347,45 g Wasser vorgelegt und aufgeheizt bis 100°C. Zum Inertisieren Stickstoff in den Reaktor einleiten. Bei Rückfluss 198,14 g Diallylamin + 20EO, mit 24,24 g Dimethylsulfat quarterniert, gelöst in 55,6 g Wasser und 58 g Acrylsäure in 5 Stunden zutropfen sowie 21,05 g einer 20 %igen Na-peroxodisulfatlösung und 14g einer 20 %igen Na-hypophosphitlösung in 5,25 Stunden. Um die Copolymerisation zu vervollständigen 1 Stunde nachpolymerisieren, anschließend kühlen und mit 50 %iger Natronlauge neutralisieren.

Beispiel 4:

In einen 1 l Glasreaktor mit Ankerrührer, Thermometer, Stickstoff-Einleitung, Rückflusskühler und Tropftrichter wird 325,63 g Wasser vorgelegt und aufgeheizt bis 100°C. Zum Inertisieren Stickstoff in den Reaktor einleiten. Bei Rückfluss 290,22 g Diallylamin + 40EO gelöst in 156,28 g Wasser und 45 g Acrylsäure in 5 Stunden zutropfen sowie 25,13 g einer 20 %igen Na-peroxodisulfatlösung und 16,72 g einer 20 %igen Na-hypophosphitlösung in 5,25 Stunden. Um die Copolymerisation zu vervollständigen 1 Stunde nachpolymerisieren, anschließend kühlen und mit 50 %iger Natronlauge neutralisieren.

Beispiel 5:

In einen 1 l Glasreaktor mit Ankerrührer, Thermometer, Stickstoff-Einleitung, Rückflusskühler und Tropftrichter wird 346,45 g Wasser vorgelegt und aufgeheizt bis 100°C. Zum Inertisieren Stickstoff in den Reaktor einleiten. Bei Rückfluss 233,15 g Diallylamin + 40EO, mit 15,01 g Dimethylsulfat quarterniert, gelöst in 62,04 g Wasser und 36 g Acrylsäure in 5 Stunden zutropfen sowie 21,3 g einer 20 %igen Na-peroxodisulfatlösung und 14,2 g einer 20 %igen Na-hypophosphitlösung in 5,25 Stunden. Um die Copolymerisation zu vervollständigen 1 Stunde nachpolymerisieren, anschließend kühlen und mit 50 %iger Natronlauge neutralisieren.

Beispiel 6:

In einen 1 l Glasreaktor mit Ankerrührer, Thermometer, Stickstoff-Einleitung, Rückflusskühler und Tropftrichter wird 281,42 g Wasser vorgelegt und aufgeheizt bis 100°C. Zum Inertisieren Stickstoff in den Reaktor einleiten. Bei Rückfluss 250,32 g Diallylamin + 40EO gelöst in 134,49 g Wasser und 38,78 g Acrylsäure mit 0,217 g Hydrochinonmonomethylether in 5 Stunden zutropfen sowie 21,71 g einer 20 %igen Na-peroxodisulfatlösung und 14,43 g einer 20 %igen Na-hypophosphitlösung in 5,25 Stunden. Um die Copolymerisation zu vervollständigen 1 Stunde nachpolymerisieren, anschließend kühlen und mit 50 %iger Natronlauge neutralisieren bis pH 6,5 - 7,0.

Beispiel 7:

In einen 1 l Glasreaktor mit Ankerrührer, Thermometer, Stickstoff-Einleitung, Rückflusskühler und Tropftrichter werden 317,43 g Wasser und 96,00 g Diallylamin + 20EO vorgelegt und aufgeheizt bis 100°C. Zum Inertisieren Stickstoff in den Reaktor einleiten. Bei Rückfluss 96,00 g Diallylamin + 20EO gelöst in 51,69 g Wasser mit 3,73 g Na-hypophosphit und 56,57 g Acrylsäure in 10 Stunden zutropfen sowie 18,65 g einer 20 %igen Na-peroxodisulfatlösung in 10,25 Stunden. Um die Copolymerisation zu vervollständigen 1 Stunde nachpolymerisieren, anschließend kühlen und mit 50 %iger Natronlauge neutralisieren bis pH 6,5 - 7,0.

Beispiel 8:

In einen 1 l Glasreaktor mit Ankerrührer, Thermometer, Stickstoff-Einleitung, Rückflusskühler und Tropftrichter werden 209,23 g Wasser und 61,98 g Diallylamin + 40EO vorgelegt und aufgeheizt bis 100°C. Zum Inertisieren Stickstoff in den Reaktor einleiten. Bei Rückfluss 61,98 g Diallylamin + 40EO gelöst in 33,37 g Wasser mit 2,15 g Na-hypophosphit und 19,21 g Acrylsäure in 10 Stunden zutropfen sowie 10,75 g einer 20 %igen Na-peroxodisulfatlösung in 10,25 Stunden. Um die Copolymerisation zu vervollständigen 1 Stunde nachpolymerisieren, anschließend kühlen und mit 50 %iger Natronlauge neutralisieren bis pH 6,5 - 7,0.

Beispiel 9:

In einen 1 l Glasreaktor mit Ankerrührer, Thermometer, Stickstoff-Einleitung, Rückflusskühler und Tropftrichter wird 26,70 g Wasser vorgelegt und aufgeheizt bis 100°C. Zum Inertisieren Stickstoff in den Reaktor einleiten. Bei Rückfluss 154,40 g Diallylamin + 20EO gelöst in 83,14 g Wasser und 45,59 g Acrylsäure in 5 Stunden zutropfen sowie 3,0g VA 086 - Azo Starter in 127,0 g Wasser gelöst und 15,0 g einer 20 %igen Na-hypophosphitlösung in 5,25 Stunden. Um die Copolymerisation zu vervollständigen 1 Stunde nachpolymerisieren, anschließend kühlen und mit 50 %iger Natronlauge neutralisieren bis pH 6,5 - 7,0.

Beispiel 10:

In einen 1 l Glasreaktor mit Ankerrührer, Thermometer, Stickstoff-Einleitung, Rückflusskühler und Tropftrichter werden 187,18 g Wasser und 58,10 g Diallylamin + 40EO vorgelegt und aufgeheizt bis 70°C. Zum Inertisieren Stickstoff in den Reaktor einleiten. Bei erreichter Temperatur 58,10 g Diallylamin + 40EO gelöst in 31,28 g Wasser mit 0,64 g Na-hypophosphit und 11,26 g Acrylsäure in 10 Stunden zutropfen sowie 12,73 g einer 15 %igen Na-peroxodisulfatlösung in 10,25 Stunden. Um die Copolymerisation zu vervollständigen 1 Stunde nachpolymerisieren, anschließend kühlen und mit 50 %iger Natronlauge neutralisieren bis pH 6,5 - 7,0.

Beispiel 11

In einen 1 l Glasreaktor mit Ankerrührer, Thermometer, Stickstoff-Einleitung, Rückflusskühler und Tropftrichter wird 210,00 g Wasser und 89,86 g Diallylamin + 40EO vorgelegt und aufgeheizt bis 70°C. Zum Inertisieren Stickstoff in den Reaktor einleiten. Bei erreichter Temperatur 17,41 g Acrylsäure, 201,5 g 50 %ige wässrige Lösung von Polyethylenglykolmethylether-methacrylat (Fa. Aldrich, Mn ca. 2080) und 1;04 g Natriumhypophosphit in 9 Stunden zutropfen sowie 13,50 g einer 23,1 %igen Na-peroxodisulfatlösung in 9,25 Stunden. Um die Copolymerisation zu vervollständigen 1 Stunde nachpolymerisieren, anschließend kühlen und mit 50 %iger Natronlauge neutralisieren bis pH 6,5 - 7,0.

Prüfverfahren für Betonfließmittel auf Basis der EN 196 bzw. DIN 18555 Teil 2:

Geräte:

- Mischer Typ 203 (Fa. Testing Bluhm und Feuerhard GmbH)
- Stoppuhr
- Laborwaage (Genauigkeit +- 1 g)
- Ausbreittisch d=300mm Typ ? (Fa. Testing Bluhm und Feuerhard GmbH)
- Setztrichter
- Tropftrichter mit Schlauchanschluss
- Löffel
- Luftporengehaltmessgerät (Fa. Testing Bluhm und Feuerhard GmbH)
- Rütteltisch Typ 2.0233 (Fa. Testing Bluhm und Feuerhard GmbH)
- Prismenform (L * B * H =16 cm * 4 cm * 4 cm)

Einsatzsstoffe: Zement: Zuschlag 1:3; Sieblinie 0/2

| | |
|---|---|
| 1500 g | Normsand CEN I - III |
| 500 g | Heidelberger Zement CEM I 32,5 R |
| 225 g | Wasser (ggf. incl. Fließmittel) |

⇒ W/Z 0,45

Fließmittel: Das Fließmittel wird ca. 1d vor der Prüfung mit 0,1-0,3% eines geeigneten Entschäumers versetzt.

Anmerkung: Der Fließmittelzusatz wird als Festsubstanz berechnet, bezogen auf den Zementanteil. Die durch das Fließmittel zugefügte Wassermenge wird bei der Berechnung der Gesamtwassermenge zur Einstellung des W/Z-Wertes berücksichtigt.

Durchführung der Prüfung

a) Herstellung des Mörtels

Die gesamte Menge der Trockenmischung (Zement + Sand) wird 1 min. mit dem Mischer Typ 203 homogen gemischt.

Anschließend wird mittels Tropftrichter die Nasskomponente[1] über einen Zeitraum von 30 sek. kontinuierlich zu dosiert.

Nach 3 Minuten Nachrührzeit ist die Herstellung des Mörtels beendet. Anschließend erfolgt die erste Messung des Ausbreitmaßes

Wasser oder Wasser/Fließmittel- Gemisch

b) Ausbreitversuch nach DIN 18555 Teil 2

Zur Bestimmung des Ausbreitmaßes ist der Setztrichter mittig auf die Glasscheibe des Ausbreitmaßtisches zu stellen, der Mörtel in zwei Schichten einzufüllen und jede Schicht durch Andrücken mit dem Löffel zu verdichten. Während des Einfüllens ist der Setztrichter mit einer Hand auf die Glasplatte zu drücken.

Der überstehende Mörtel ist abzustreichen und die freie Fläche des Ausbreitmaßtisches zu reinigen. Anschließend ist der Setztrichter langsam senkrecht nach oben zu ziehen und der Mörtel auf der Glasplatte mit 15 Hubschlägen auszubreiten.

Nun ist der Durchmesser des ausgebreiteten Mörtels in zwei rechtwinklig zueinander stehenden Richtungen zu messen. Das Ergebnis ist in cm auf 0,5 cm als arithmetisches Mittel anzugeben.

Die Bestimmung erfolgt nach 5, 30, 60, und 90 Minuten. Vor jeder Messung wird der Mörtel kurz aufgerührt.

c) Luftgehalt des Mörtels in Anlehnung an DIN 18555 Teil 2

Der Luftgehalt des Frischmörtels wird mit einem justierten Prüfgerät von $1 dm^3$ Inhalt nach dem Druckausgleichsverfahren gemessen.

Dazu füllt man den 1 l Behälter des Luftporengehaltmeßgerätes mit Mörtel, während der Mörtel 60 s lang auf dem Rütteltisch verdichtet wird.

Anschließend wird das Oberteil des Prüfgerätes auf den gesäuberten Schliffrand des Behälters gesetzt und das Gerät verschlossen.

Das noch freie Volumen des Gerätes wird mit Wasser gefüllt. In der Kammer wird ein definierter Druck erzeugt. Nach erfolgtem Druckausgleich wird der Luftporengehalt auf der am Oberteil angebrachten Skala direkt abgelesen.

Der Luftporengehalt, ausgedrückt als Volumenanteil in %, wird mit einer Messgenauigkeit von 0,1 % angegeben.

| | Feststoff [%] | pH-Wert | K-Wert [1% in $H_2O$] | Mn Zahlenmittel | Mw Gewichtsmittel | Fließmittel Dosierung | Ausbreitmaß 5 Min. | 30 Min. | 60 Min. | 90 Min. |
|---|---|---|---|---|---|---|---|---|---|---|
| Bsp. 1. | 38,8 | 6,9 | 24,9 | 5400 | 19700 | 0,20% | 17,5 | 16,4 | 15,3 | 14,3 |
| Bsp. 2. | 44,2 | 6,9 | 26,8 | 5900 | 24500 | 0,20% | 17,1 | 15,9 | 15,8 | 14,4 |
| Bsp. 3. | 38,6 | 6,7 | 22,9 | 6000 | 35900 | 0,30% | 17,0 | 15,3 | 14,5 | 14,6 |
| Bsp. 4. | 39,4 | 6,8 | 29,5 | 5200 | 19600 | 0,30% | 18,1 | 18,1 | 17,2 | 16,7 |
| Bsp. 5. | 41,7 | 7,0 | 24,2 | 4100 | 12200 | 0,30% | 16,1 | 15,2 | 13,5 | 13,7 |
| Bsp. 6. | 39,5 | 6,9 | 27,8 | 8200 | 30100 | 0,30% | 18,5 | 17,9 | 17,2 | 16,2 |
| Bsp. 7. | 38,5 | 6,8 | 26,7 | 5900 | 19600 | 0,20% | 16,7 | 15,6 | 13,8 | - |
| Bsp. 8. | 36,2 | 7,0 | 27,3 | 5300 | 15000 | 0,20% | 16,6 | 15,8 | 13,4 | - |
| Bsp. 9. | 38,2 | 6,7 | 30,2 | 6800 | 21500 | 0,20% | 15,6 | 14,9 | 13,5 | - |
| Bsp. 10. | 35,5 | 6,9 | 32,6 | 6400 | 33000 | 0,20% | 18,1 | 16,8 | 15,4 | 14,7 |
| Bsp. 11 | 39,0 | 6,7 | 42,1 | 9100 | 84000 | 0,20% | 15,6 | 15,6 | 14,3 | 13,0 |

## Patentansprüche

1. Wasserlösliche oder in Wasser dispergierbare Polymerisate enthaltend

   (a) mindestens ein alkoxyliertes Diallylaminderivat (Monomer A)
   (b) mindestens eine ethylenisch ungesättigte Mono- oder Dicarbonsäure, deren Anhydride oder Gemische davon (Monomer B) sowie
   (c) gegebenenfalls eines oder mehrere weitere ethylenisch ungesättigte Monomere C.

2. Polymerisate gemäß Anspruch 1, **dadurch gekennzeichnet, dass** als Monomer A mindestens eine Verbindung der allgemeinen Formel 1 eingesetzt wird

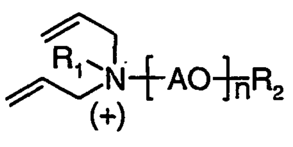 (I)

   wobei

   AO ein $C_1$-$C_{12}$-Alkylenoxid, Styroloxid oder ein Gemisch von zwei oder mehr Arten hiervon bedeutet, wobei die zwei oder mehr Arten entweder in Blockform oder in statistischer Form aneinander gefügt sein können,
   n eine ganze Zahl von 2 bis 200
   $R_1$ Wasserstoff, $C_1$-$C_{20}$-Alkyl, $C_5$-$C_{10}$-Cycloalkyl oder einen gegebenenfalls substituierten Benzylrest bedeutet und
   $R_2$ Wasserstoff, $C_1$-$C_{30}$-Alkyl, $C_5$-$C_8$-Cycloalkyl, $C_6$-$C_{20}$-Aryl, $C_1$-$C_{30}$-Alkanoyl, $C_7$-$C_{21}$-Aroyl, Schwefelsäure (halb)ester, Phosphorsäureester, NR'R", NR'R"R"'$^{3+}$bedeutet und
   R', R", R"' jeweils unabhängig voneinander gleich oder verschieden sein können und Wasserstoff, einen geradkettig oder verzweigten $C_1$-$C_{20}$-Alkyl- oder einen geradkettig oder verzweigten $C_1$-$C_{20}$-Hydroxyalkylrest

bedeuten.

3. Polymerisate gemäß Anspruch 1, **dadurch gekennzeichnet, dass** als Monomer B mindestens eine Verbindung der allgemeinen Formel II oder deren Anhydride eingesetzt werden

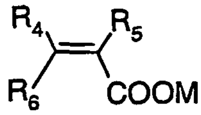

(II)

wobei

$R_4$, $R_5$ unabhängig voneinander entweder gleich oder verschieden sein können und Wasserstoff oder $C_1$-$C_6$-Alkyl bedeuten,
$R_6$ Wasserstoff, $C_1$-$C_6$-Alkyl oder eine Gruppe COOM bedeutet und
M Wasserstoff, ein ein- oder zweiwertiges Metallion, Ammonium oder ein organisches Ammoniumion bedeutet.

4. Polymerisate gemäß einem der Ansprüche 1 bis 3, **dadurch gekennzeichnet, dass** das molare Verhältnis der Monomere A zu den Monomeren B 1 :1 bis 1 : 6 beträgt.

5. Polymerisate gemäß einem der Ansprüche 1 bis 4, **dadurch gekennzeichnet, dass** das molare Verhältnis der Monomere A zu den Monomeren B 1 : 2 bis 1 : 5 beträgt.

6. Polymerisate gemäß einem der Ansprüche 1 bis 5, **dadurch gekennzeichnet, dass** das gewichtsmittlere Molekulargewicht $M_w$ der Polymerisate im Bereich von 1000 bis 100 000 liegt.

7. Polymerisate gemäß einem der Ansprüche 1 bis 6, **dadurch gekennzeichnet, dass** sie einen K-Wert von 20 bis 50 aufweisen.

8. Polymerisate gemäß einem der Ansprüche 1 bis 7, erhältlich durch radikalische Polymerisation der Monomere A mit Monomeren B sowie gegebenenfalls weiteren Monomeren C.

9. Verwendung der Polymerisate gemäß einem der Ansprüche 1 bis 8, als Additiv in mineralischen Baustoffen, Waschmitteln oder in kosmetischen Mitteln.

10. Verwendung der Polymerisate gemäß Anspruch 9, als Additive in mineralischen Baustoffen.

11. Verwendung gemäß einem der Ansprüche 9 oder 10, wobei die zu polymerisierende Monomerenmischung

1-70 Mol.-% Monomer A
10-99 Mol.-% Monomer B und
0-50 Mol.-% Monomer C enthält.

12. Zement-Dispergiermittel, enthaltend mindestens ein Polymerisat gemäß einem der Ansprüche 1 bis 8.

13. Mineralischer Baustoff enthaltend Zement, Wasser, wenigstens ein Polymerisat gemäß einem der Ansprüche 1 bis 8, sowie weitere übliche Zuschlagstoffe.

## Claims

1. A water-soluble or water-dispersible polymer comprising

(a) at least one alkoxylated diallylamine derivative (monomer A),
(b) at least one ethylenically unsaturated mono- or dicarboxylic acid, the anhydrides thereof or mixtures thereof (monomer B) and
(c) if required, one or more further ethylenically unsaturated monomers C.

2. The polymer according to claim 1, wherein at least one compound of the formula I

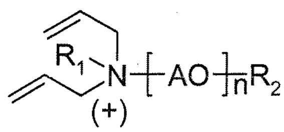

(I)

where

AO is a $C_1$-$C_{12}$-alkylene oxide, styrene oxide or a mixture of two or more types thereof, it being possible for the two or more types to be attached to one another in block form or in random form,
n is an integer from 2 to 200
$R_1$ is hydrogen, $C_1$-$C_{20}$-alkyl, $C_5$-$C_{10}$-cycloalkyl or an unsubstituted or substituted benzyl radical and
$R_2$ is hydrogen, $C_1$-$C_{30}$-alkyl, $C_5$-$C_8$-cycloalkyl, $C_6$-$C_{20}$-aryl, $C_1$-$C_{30}$-alkanoyl, $C_7$-$C_{21}$-aroyl, a sulfuric(mono) ester, a phosphoric ester, NR'R" or NR'R"R"'$^{3+}$ and
R', R" and R"', in each case independently of one another, may be identical or different and are hydrogen, a straight-chain or branched $C_1$-$C_{20}$-alkyl radical or a straight-chain or branched $C_1$-$C_{20}$-hydroxyalkyl radical,

is used as monomer A.

3. The polymer according to claim 1, wherein at least one compound of the formula II or the anhydrides thereof

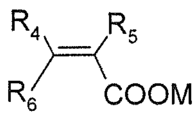

(II)

where

$R_4$ and $R_5$, independently of one another, may be either identical or different and are hydrogen or $C_1$-$C_6$-alkyl,
$R_6$ is hydrogen, $C_1$-$C_6$-alkyl or a COOM group and
M is hydrogen, a monovalent or divalent metal ion, ammonium or an organic ammonium ion,

is or are used as monomer B.

4. The polymer according to any of claims 1 to 3, wherein the molar ratio of the monomers A to the monomers B is from 1 : 1 to 1 : 6.

5. The polymer according to any of claims 1 to 4, wherein the molar ratio of the monomers A to the monomers B is from 1 : 2 to 1 : 5.

6. The polymer according to any of claims 1 to 5, wherein the weight average molecular weight $M_w$ of the polymers is from 1000 to 100 000.

7. The polymer according to any of claims 1 to 6, which has a K value of from 20 to 50.

8. The polymer according to any of claims 1 to 7, obtainable by free radical polymerization of the monomers A with monomers B and, if required, further monomers C.

9. The use of a polymer according to any of claims 1 to 8 as an additive in mineral building materials, in detergents or in cosmetic compositions.

10. The use of a polymer according to claim 9 as an additive in mineral building materials.

**11.** The use according to either of claims 9 and 10, the monomer mixture to be polymerized containing

1-70 mol% of monomer A,
10-99 mol% of monomer B and
0-50 mol% of monomer C.

**12.** A cement dispersant comprising at least one polymer according to any of claims 1 to 8.

**13.** A mineral building material comprising cement, water, at least one polymer according to any of claims 1 to 8 and further conventional additives.

**Revendications**

**1.** Polymères solubles ou dispersibles dans l'eau contenant :

(a) au moins un dérivé alcoxylé de diallylamine (monomère A),
(b) au moins un acide mono- ou dicarboxylique à insaturation éthylénique, leurs anhydrides ou leurs mélanges (monomère B) ainsi que
(c) éventuellement un ou plusieurs autres monomères C à insaturation éthylénique.

**2.** Polymères selon la revendication 1, **caractérisés en ce que** l'on utilise comme monomère A au moins un composé de formule générale I :

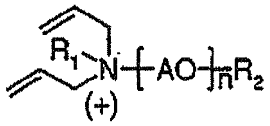

(I)

dans laquelle :

AO est un oxyde d' alkylène en $C_1$-$C_{12}$, un oxyde de styrène ou un mélange de deux ou plusieurs de ces types, les deux types ou plus pouvant être assemblés l'un avec l'autre sous forme de séquence ou sous forme statistique,
n est un nombre entier de 2 à 200,
$R_1$ représente de l'hydrogène, un groupement alkyle en $C_1$-$C_{20}$, cycloalkyle en $C_5$-$C_{10}$ ou un radical benzyle éventuellement substitué et
$R_2$ représente de l'hydrogène, un groupement alkyle en $C_1$-$C_{30}$, cycloalkyle en $C_5$-$C_8$, aryle en $C_6$-$C_{20}$, alcanoyle en $C_1$-$C_{30}$, aroyle en $C_7$-$C_{21}$, (semi-) ester d'acide sulfurique, ester d'acide phosphorique, NR'R'', NR'R''R'''$^{3+}$ et R', R'', R''' peuvent être, indépendamment l'un de l'autre, respectivement identiques ou différents et représenter de l'hydrogène, un radical alkyle en $C_1$-$C_{20}$ linéaire ou ramifié ou un radical hydroxyalkyle en $C_1$-$C_{20}$ linéaire ou ramifié.

**3.** Polymères selon la revendication 1, **caractérisés en ce que** l'on utilise comme monomère B au moins un composé de formule générale II ou ses anhydrides :

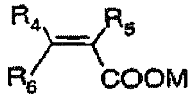

(II)

dans laquelle :

$R_4$, $R_5$ peuvent, indépendamment l'un de l'autre, être identiques ou différents et représenter de l'hydrogène ou un groupement alkyle en $C_1$-$C_6$,
$R_6$ représente de l'hydrogène, un groupement alkyle en $C_1$-$C_6$ ou un groupement COOM et

M représente de l'hydrogène, un ion métallique monovalent ou divalent, de l'ammonium ou un ion d'ammonium organique.

4. Polymères selon l'une quelconque des revendications 1 à 3, **caractérisés en ce que** le rapport molaire des monomères A aux monomères B est de 1:1 à 1:6.

5. Polymères selon l'une quelconque des revendications 1 à 4, **caractérisés en ce que** le rapport molaire des monomères A aux monomères B est de 1:2 à 1:5.

6. Polymères selon l'une quelconque des revendications 1 à 5, **caractérisés en ce que** le poids moléculaire moyen en poids Mw des polymères se situe dans la plage de 1000 à 100 000.

7. Polymères selon l'une quelconque des revendications 1 à 6, **caractérisés en ce qu'**ils présentent une valeur K de 20 à 50.

8. Polymères selon l'une quelconque des revendications 1 à 7, que l'on peut obtenir par polymérisation radicalaire des monomères A avec des monomères B ainsi qu'éventuellement d'autres monomères C.

9. Utilisation des polymères selon l'une quelconque des revendications 1 à 8, comme additifs dans des matériaux de construction minéraux, dans des agents de lavage ou dans des agents cosmétiques.

10. Utilisation des polymères selon la revendication 9 comme additifs dans des matériaux de construction minéraux.

11. Utilisation selon l'une quelconque des revendications 9 ou 10, dans laquelle le mélange de monomères à polymériser contient 1 à 70% en mole de monomère A, 10 à 99% en mole de monomère B et 0 à 50% en mole de monomère C.

12. Agent dispersant de ciment contenant au moins un polymère selon l'une quelconque des revendications 1 à 8.

13. Matériau de construction minéral contenant du ciment, de l'eau, au moins un polymère selon l'une quelconque des revendications 1 à 8 ainsi que d'autres additifs habituels.